# EUROPEAN PATENT APPLICATION

(11) **EP 4 620 667 A1**
(43) Date of publication of application: **24.09.2025**
(21) Application number: 25164829.1
(22) Date of filing: 19.03.2025
(51) Int. Cl.: B32B 5/02, A61F 13/15, A61F 13/514, A61F 13/51, B32B 5/26, D06M 10/00, D06M 10/10, D06M 13/224, D06M 14/26, D06M 14/28, D06M 15/285, D06M 15/356, D06M 15/643, D06M 15/65, C08G 77/20, C09D 183/06

(54) **BARRIER FABRICS WITH DESIRABLE AIR PERMEABILITY**

(30) Priority: 20.03.2024 CN 202410322659
(71) Applicant: NANHAI NANXIN NON-WOVEN CO. LTD., Foshan City, Guangdong (CN)
(72) Inventor: WANG, Lei, MOORESVILLE, 28117 (US); BISHOP, Nyle, MOORESVILLE, 28117 (US); ZHANG, Tianlei, SUZHOU, 215126 (CN); JIN, Yongji, SUZHOU, 215126 (CN); GAO, Junying, SUZHOU, 215126 (CN)
(74) Representative: Groth & Co. KB

(57) **Abstract**

A barrier fabric suitable as a backsheet for an underpad configured for a low air loss bed, in which the barrier fabric comprises a first nonwoven fabric including outermost spunbond layers and two or more fine-fiber containing nonwoven layers. Each of the nonwoven layers being treated with a non-fluorinated barrier coating (NFBC).

## Description

### TECHNICAL FIELD

Embodiments of the presently-disclosed invention relate generally to barrier fabrics comprising a multilayer nonwoven fabric including outer spunbond layers with a plurality of inner fine fiber-containing layers, in which each individual layer may be treated with a non-fluorinated barrier coating (NFBC). The barrier fabrics may be provided as a backsheet, such as for an underpad or a personal hygiene article, having desirable liquid barrier properties in relation to low surface tension fluids (e.g., alcohols and blood) while also having a desirably high air permeability.

### BACKGROUND

Low air loss beds are prescribed for patients that are admitted with pressure sores, patients at risk of developing pressure sores, or other skin ailments. These beds may be designed with multiple air chambers that alternatively fill with air and deflate over time so that pressure of a given area of a patient's skim is varied over time in an attempt to treat and/or prevent pressure sores or the like. Patients utilizing low air loss beds often require the use of an underpad, which is placed between the bedsheets and the mattress. Underpads are intending to help prevent bodily fluids from penetrating into the mattress..

Underpads are generally formed from a topsheet, an absorbent core, and a backsheet. A common backsheet in current underpads utilize a microporous film/spunbond structure. This approach, however, does not provide a level of air permeability as high as currently desired. For instance, an increase in air flow (e.g., high air permeability through the backsheet and underpad) is currently considered to be important for the effective use of low air loss beds to facilitate the prevention and/or treatment of pressure sores or the like. Additionally, the underpads should also be strong enough to withstand the ongoing repositioning of a patient, which may weigh up to 350 pounds. Still further yet, the underpads would also desirably provide a hydrostatic head of greater than 100 mbar and desirable alcohol repellency to provide a liquid barrier to human urine (or other bodily fluids) without penetration into the mattress.

Accordingly, there remains a need in the art for a barrier fabric including a combination of liquid barrier properties and high air permeability suitable as a backsheet, for example, for an underpad configured for use with a low air loss bed.

### SUMMARY OF INVENTION

One or more embodiments of the invention may address one or more of the aforementioned problems. Certain embodiments according to the invention provide barrier fabric comprising a first nonwoven fabric. The first nonwoven fabric may include a first outermost spunbond layer including a first plurality of continuous spunbond fibers and a second outermost spunbond layer including a second plurality of continuous spunbond fibers. The first nonwoven fabric comprising a plurality of inner fine fiber-containing nonwoven layers including a first fine fiber-containing layer including a first plurality of fine fibers and a second fine fiber-containing layer including a second plurality of fine fibers, in which the plurality of inner fine fiber-containing nonwoven layers are located directly or indirectly between the first outermost spunbond layer and the second outermost spunbond layer. A non-fluorinated barrier coating (NFBC) may be located on each of the first outermost spunbond layer, the second outermost spunbond layer, the first fine fiber-containing layer, and the second fine fiber-containing layer.

In another aspect, the present invention provides an underpad for a low air loss bed. The underpad may comprise a backsheet including or consisting of a barrier fabric according to any of those described and disclosed herein, a liquid permeable topsheet, an absorbent core located between the backsheet and the liquid permeable topsheet.

In another aspect, the present invention provides a method of forming a barrier fabric, in which the method may include the following steps: (i) providing or forming a first outermost spunbond layer including a first plurality of continuous spunbond fibers; (ii) providing or forming a second outermost spunbond layer including a second plurality of continuous spunbond fibers; (iii) providing or forming a plurality of inner fine fiber-containing nonwoven layers including a first fine fiber-containing layer including a first plurality of fine fibers and a second fine fiber-containing layer including a second plurality of fine fibers; (iv) positioning the plurality of inner fine fiber-containing nonwoven layers directly or indirectly between the first outermost spunbond layer and the second outermost spunbond layer; and (v) topically applying a non-fluorinated barrier coating (NFBC) composition on each of the first outermost spunbond layer, the second outermost spunbond layer, the first fine fiber-containing layer, and the second fine fiber-containing layer.

### BRIEF DESCRIPTION OF THE DRAWING(S)

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. Like numbers refer to like elements throughout, and wherein:
Figure 1 illustrates a cross-sectional view of a barrier fabric according to certain embodiments of the invention;
Figure 2 illustrates a cross-sectional view of another barrier fabric according to certain embodiments of the invention; and

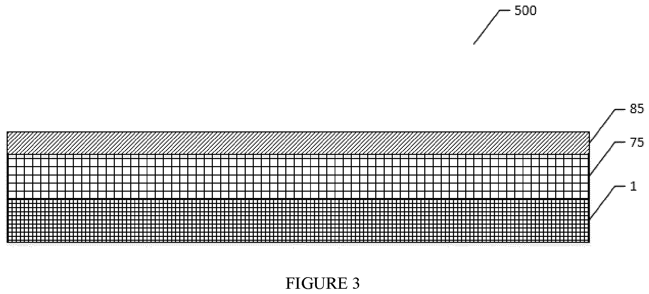

Figure 3 illustrates a cross-sectional view of an underpad for use with a low air loss bed in accordance with certain embodiments of the invention.

### DETAILED DESCRIPTION

The invention now will be described more fully hereinafter with reference to the accompanying drawings, in which some, but not all embodiments of the invention are shown. Indeed, this invention may be embodied in many different forms and should not be construed as limited to the embodiments set forth herein; rather, these embodiments are provided so that this disclosure will satisfy applicable legal requirements. As used in the specification, and in the appended claims, the singular forms "a", "an", "the", include plural referents unless the context clearly dictates otherwise.

The presently-disclosed invention relates generally to barrier fabrics that may be used as a backsheet for an underpad or a variety of personal hygiene article (e.g., a diaper). In this regard, the backsheet may be used to replace the traditional use of a film-based backsheet. In this regard, an underpad incorporating a backsheet as described and disclosed herein may be devoid of a film. In accordance with certain embodiments of the invention, the backsheet may include a first nonwoven fabric including multiple fine fiber-containing nonwoven layers (e.g., meltblown layers) in which each of these layers being treated or coated with a low surface tension fluoro-free chemical coating. For example, the non-fluorinated barrier coating (NFBC) applied to the first nonwoven fabric may provide a performance comparable to C6 fluorine chemical treated materials to prevent lower surface tension fluid penetration such as blood (Synthetic Blood used in ASTM F1670M ), and/or 0.9% saline solution penetration in a manner similar to or better than breathable films. As such, certain embodiments of the invention provide a barrier fabric that may be used as a backsheet, which may replace typical film-based backsheets) in underpads, diapers, feminine hygiene articles, and adult incontinence diapers.

The NFBC utilizes lower surface tension chemicals that are devoid of fluorine atoms to coat individual nonwoven layers (e.g., silicone rubber has a reported surface energy value from 19-22 mJ/m²). In accordance with certain embodiments of the invention, the individual nonwoven layers if the barrier fabric may be coated with chemicals that are devoid of fluorine atoms, which may replace the reliance on fluoro-chemicals while achieving similar or improved barrier repellency performance (e.g., prevent the penetration of low surface tension fluids through the fabric). In accordance with certain embodiments of the invention, for example, the fabrics including the NFBC exhibit performance comparable to or better than C6 fluorine chemical treated materials with respect to preventing lower surface tension fluid penetration and also passing the blood drop test with synthetic blood in accordance with ASTM F1670M (e.g., 15 minutes of droplet exposure without blood penetration). By way of example only, polypropylene may have a surface tension of 30.5 mJ/m² and polyethylene may have a surface tension of 31.6 mJ/m². Coating or covering a fabric formed from polypropylene or polyethylene, for example, with the NFBC may reduce the surface tension of the treated surface due to the NFBC, which may render the treated surface resistant to low surface tension fluid penetration. In accordance with certain embodiments of the invention, the barrier fabric may be devoid of fluorine atoms.

In accordance with certain embodiments of the invention, the barrier fabric may optionally be made by any method known, such as those described and disclosed herein. Moreover, the barrier fabric may be made from a broad choice of polymeric materials, such as polyolefins (e.g., polypropylene, polyethylene, copolymers thereof, etc.), polyesters, polyamides, natural fibers (e.g., cotton, etc.), and cellulosic fibers (e.g., rayon, wood fibers, etc.). In accordance with certain embodiments of the invention, for example, the barrier fabric comprises a first nonwoven fabric comprising a polyolefin thermoplastic polymer. For example, the first nonwoven fabric may comprise a polypropylene (e.g., polypropylene being defined broadly and includes copolymers and blends containing a polypropylene). In accordance with certain embodiments of the invention, the first nonwoven fabric may comprise a polyethylene (e.g., polyethylene monocomponent fibers, bi-component fibers including a polyethylene component, flash spun polyethylene fibers, etc.).

In accordance with certain embodiments of the invention, the barrier fabric may comprise a first nonwoven fabric including continuous fibers (e.g., spunbond fibers), staple fibers, fine fibers (e.g., defined broadly to include melt-blown, melt-film fibrillated, electrospun, etc.). As noted above, certain embodiments of the invention may comprise a barrier fabric including a first nonwoven fabric comprising a layer of cellulosic fibers (e.g., wood pulp) and a layer of synthetic fibers (e.g., thermoplastic polymer) mechanically entangled together (e.g., hydroentangled together). In accordance with certain embodiments of the invention, the first nonwoven fabric may comprise continuous fibers (e.g., spunbond fibers) and fine fibers (e.g., meltblown fibers), such as fabrics having a spunbond-meltblown-spunbond (SMS), such as a SMS or SSMMS structure, where one or several layers of meltblown fibers are sandwiched in between layers of continuous fibers.

The terms "substantial" or "substantially" may encompass the whole amount as specified, according to certain embodiments of the invention, or largely but not the whole amount specified (e.g., 95%, 96%, 97%, 98%, or 99% of the whole amount specified) according to other embodiments of the invention.

The terms "polymer" or "polymeric", as used interchangeably herein, may comprise homopolymers, copolymers, such as, for example, block, graft, random, and alternating copolymers, terpolymers, etc., and blends and modifications thereof. Furthermore, unless otherwise specifically limited, the term "polymer" or "polymeric" shall include all possible structural isomers; stereoisomers including, without limitation, geometric isomers, optical isomers or enantionmers; and/or any chiral molecular configuration of such polymer or polymeric material. These configurations include, but are not limited to, isotactic, syndiotactic, and atactic configurations of such polymer or polymeric material. The term "polymer" or "polymeric" shall also include polymers made from various catalyst systems including, without limitation, the Ziegler-Natta catalyst system and the metallocene/single-site catalyst system. The term "polymer" or "polymeric" shall also include, in according to certain embodiments of the invention, polymers produced by fermentation process or biosourced.

The terms "nonwoven" and "nonwoven web", as used herein, may comprise a web having a structure of individual fibers, filaments, and/or threads that are interlaid but not in an identifiable repeating manner as in a knitted or woven fabric. Nonwoven fabrics or webs, according to certain embodiments of the invention, may be formed by any process conventionally known in the art such as, for example, meltblowing processes, spunbonding processes, needle-punching, hydroentangling, air-laid, and bonded carded web processes. A "nonwoven web", as used herein, may comprise a plurality of individual fibers that have not been subjected to a consolidating process. In certain instances, the "nonwoven web" may comprises a plurality of layers, such as one or more spunbond layers and/or one or more meltblown layers. For instance, a "nonwoven web" may comprises a spunbond-meltblown-spunbond structure.

The terms "fabric" and "nonwoven fabric", as used herein, may comprise a web of fibers in which a plurality of the fibers are mechanically entangled or interconnected, fused together, and/or chemically bonded together. For example, a nonwoven web of individually laid fibers may be subjected to a bonding or consolidation process to bond at least a portion of the individually fibers together to form a coherent (e.g., united) web of interconnected fibers.

The term "consolidated" and "consolidation", as used herein, may comprise the bringing together of at least a portion of the fibers of a nonwoven web into closer proximity or attachment there-between (e.g., thermally fused together, chemically bonded together, and/or mechanically entangled together) to form a bonding site, or bonding sites, which function to increase the resistance to external forces (e.g., abrasion and tensile forces), as compared to the unconsolidated web. The bonding site or bonding sites, for example, may comprise a discrete or localized region of the web material that has been softened or melted and optionally subsequently or simultaneously compressed to form a discrete or localized deformation in the web material. Furthermore, the term "consolidated" may comprise an entire nonwoven web that has been processed such that at least a portion of the fibers are brought into closer proximity or attachment there-between (e.g., thermally fused together, chemically bonded together, and/or mechanically entangled together), such as by thermal bonding or mechanical entanglement (e.g., hydroentanglement) as merely a few examples. Furthermore, the term "consolidated" and "consolidation" may comprise the bonding by means of a through-air-bonding operation. The term "through-air bonded" and "though-air-bonding", as used herein, may comprise a nonwoven web consolidated by a bonding process in which hot air is used to fuse the fibers at the surface of the web and optionally internally within the web. By way of example only, hot air can either be blown through the web in a conveyorized oven or sucked through the web as it passes over a porous drum as a vacuum is developed. The temperature of and the rate of hot air are parameters that may determine the level or the extent of bonding in nonwoven web. In accordance with certain embodiments of the invention, the temperature of the hot air may be high enough to melt, induce flowing, and/or fuse the a plurality of fibers having a lower melting point temperature or onset of lower melting point temperature (e.g., amorphous fibers) to a plurality of fibers having a higher melting point temperature or onset of lower melting point temperature (e.g., semicrystalline or crystalline fibers). Such a web may be considered a "consolidated nonwoven", "nonwoven fabric" or simply as a "fabric" according to certain embodiments of the invention.

The term "layer", as used herein, may comprise a generally recognizable combination of similar material types and/or functions existing in the X-Y plane.

The term "spunbond", as used herein, may comprise fibers which are formed by extruding molten thermoplastic material as filaments from a plurality of fine, usually circular, capillaries of a spinneret with the diameter of the extruded filaments then being rapidly reduced. According to an embodiment of the invention, spunbond fibers are generally not tacky when they are deposited onto a collecting surface and may be generally continuous as disclosed and described herein. It is noted that the spunbond used in certain composites of the invention may include a nonwoven described in the literature as SPINLACE^{®}.

As used herein, the term "continuous fibers" refers to fibers which are not cut from their original length prior to being formed into a nonwoven web or nonwoven fabric. Continuous fibers may have average lengths ranging from greater than about 15 centimeters to more than one meter, and up to the length of the web or fabric being formed. For example, a continuous fiber, as used herein, may comprise a fiber in which the length of the fiber is at least 1,000 times larger than the average diameter of the fiber, such as the length of the fiber being at least about 5,000, 10,000, 50,000, or 100,000 times larger than the average diameter of the fiber.

The term "meltblown", as used herein, may comprise fibers formed by extruding a molten thermoplastic material through a plurality of fine die capillaries as molten threads or filaments into converging high velocity, usually hot, gas (e.g. air) streams which attenuate the filaments of molten thermoplastic material to reduce their diameter, which may be to microfiber diameter, according to certain embodiments of the invention. According to an embodiment of the invention, the die capillaries may be circular. Thereafter, the meltblown fibers are carried by the high velocity gas stream and are deposited on a collecting surface to form a web of randomly disbursed meltblown fibers. Meltblown fibers may comprise microfibers which may be continuous or discontinuous and are generally tacky when deposited onto a collecting surface. Meltblown fibers, however, are shorter in length than those of spunbond fibers.

The term "melt fibrillation", as used herein, may comprise a general class of making fibers defined in that one or more polymers are molten and may be extruded into many possible configurations (e.g. co-extrusion, homogeneous or bicomponent films or filaments) and then fibrillated or fiberized into a plurality of individual filaments for the formation of melt-fibrillated fibers. Non limiting examples of melt-fibrillation methods may include melt blowing, melt fiber bursting, and melt film fibrillation. The term "melt-film fibrillation", as used herein, may comprise a method in which a melt film is produced from a melt and then a fluid is used to form fibers (e.g., melt-film fibrillated fibers) from the melt film. Examples include U.S. Pat. Nos. 6,315,806, 5,183,670, 4,536,361, 6,382,526, 6,520,425, and 6,695,992, in which the contents of each are incorporated by reference herein to the extent that such disclosures are consistent with the present disclosure. Additional examples include U.S. Pat. Nos. 7,628,941, 7,722,347, 7,666,343, 7,931,457, 8,512,626, and 8,962,501, which describe the Arium^{™} melt-film fibrillation process for producing melt-film fibrillated fibers (e.g., having sub-micron fibers).

As used herein, the term "aspect ratio", comprise a ratio of the length of the major axis to the length of the minor axis of the cross-section of the fiber in question.

The term "multi-component fibers", as used herein, may comprise fibers formed from at least two different polymeric materials or compositions (e.g., two or more) extruded from separate extruders but spun together to form one fiber. The term "bi-component fibers", as used herein, may comprise fibers formed from two different polymeric materials or compositions extruded from separate extruders but spun together to form one fiber. The polymeric materials or polymers are arranged in a substantially constant position in distinct zones across the cross-section of the multi-component fibers and extend continuously along the length of the multi-component fibers. The configuration of such a multi-component fiber may be, for example, a sheath/core arrangement wherein one polymer is surrounded by another, an eccentric sheath/core arrangement, a side-by-side arrangement, a pie arrangement, or an "islands-in-the-sea" arrangement, each as is known in the art of multicomponent, including bicomponent, fibers.

The term "fluorochemical", as used herein, may comprise any of various chemical compounds containing fluorine, particularly organic compounds (e.g., fluorocarbons such as perfluoroalkanes) in which fluorine has replaced a large proportion of the hydrogen attached to the carbons. Fluorochemicals may exhibit low surface tension and low viscosity and are extremely stable due to the strength of the carbon-fluorine bond. Fluorochemicals are not miscible with most organic solvents.

The term "dry basis", as used herein may comprise the calculation or measurement of a weight percentage in which the presence of water and/or other solvents (e.g., alcohols) are ignored or excluded for purposes of the calculation or measurement. Weight percentages may frequently be measured on a dry basis to remove the effects of evaporation and/or condensation which may happen naturally throughout the useful life of a composition or article.

The term "cellulosic fiber", as used herein, may comprise fibers derived from hardwood trees, softwood trees, or a combination of hardwood and softwood trees prepared for use in, for example, a papermaking furnish and/or fluff pulp furnish by any known suitable digestion, refining, and bleaching operations. The cellulosic fibers may comprise recycled fibers and/or virgin fibers. Recycled fibers differ from virgin fibers in that the fibers have gone through the drying process at least once. In certain embodiments, at least a portion of the cellulosic fibers may be provided from non-woody herbaceous plants including, but not limited to, kenaf, cotton, hemp, jute, flax, sisal, or abaca. Cellulosic fibers may, in certain embodiments of the invention, comprise either bleached or unbleached pulp fiber such as high yield pulps and/or mechanical pulps such as thermo-mechanical pulping (TMP), chemical-mechanical pulp (CMP), and bleached chemical-thermo-mechanical pulp BCTMP. In this regard, the term "pulp", as used herein, may comprise cellulose that has been subjected to processing treatments, such as thermal, chemical, and/or mechanical treatments. Cellulosic fibers, according to certain embodiments of the invention, may comprise one or more pulp materials.

All whole number end points disclosed herein that can create a smaller range within a given range disclosed herein are within the scope of certain embodiments of the invention. By way of example, a disclosure of from about 10 to about 15 includes the disclosure of intermediate ranges, for example, of: from about 10 to about 11; from about 10 to about 12; from about 13 to about 15; from about 14 to about 15; etc. Moreover, all single decimal (e.g., numbers reported to the nearest tenth) end points that can create a smaller range within a given range disclosed herein are within the scope of certain embodiments of the invention. By way of example, a disclosure of from about 1.5 to about 2.0 includes the disclosure of intermediate ranges, for example, of: from about 1.5 to about 1.6; from about 1.5 to about 1.7; from about 1.7 to about 1.8; etc.

Certain embodiments according to the invention provide barrier fabric comprising a first nonwoven fabric. The first nonwoven fabric may include a first outermost spunbond layer including a first plurality of continuous spunbond fibers and a second outermost spunbond layer including a second plurality of continuous spunbond fibers. The first nonwoven fabric comprising a plurality of inner fine fiber-containing nonwoven layers including a first fine fiber-containing layer including a first plurality of fine fibers and a second fine fiber-containing layer including a second plurality of fine fibers, in which the plurality of inner fine fiber-containing nonwoven layers are located directly or indirectly between the first outermost spunbond layer and the second outermost spunbond layer. A non-fluorinated barrier coating (NFBC) may be located on each of the first outermost spunbond layer, the second outermost spunbond layer, the first fine fiber-containing layer, and the second fine fiber-containing layer.

In accordance with certain embodiments of the invention, the NFBC comprises an alcohol repellant that is devoid of fluorine atoms. The NFBC, for example, may comprise an alcohol repellant comprising an acrylic-functional polymer including an alkyl silane methacrylate group. By way of example, the acrylic-functional polymer may be anionic, cationic, or non-ionic. In accordance with certain embodiments of the invention, the alkyl portion(s) of the acrylic-functional polymer may independently comprise from 1 to about 20 carbon atoms, such as at least about any of the following: 1, 2, 3, 4, 5, 6, 7, 8, 9, and 10 carbon atoms, and/or at most about any of the following: 20, 19, 18, 17, 16, 15, 14, 13, 12, 11, and 10 carbon atoms.

In accordance with certain embodiments of the invention, the NFBC comprises a cured siliconized coating. For example, the cured siliconized coating may comprise a network of radiation cured silicones. The network of radiation cured silicones may be cured and/or bonded to the fibrous structure via one of more radically curing functionalities. For example, the network of radiation cured silicones may comprise the reaction product of one or more silicone acrylate oligomers or polymers or one or more epoxy silicone oligomers or polymers.

In accordance with certain embodiments of the invention, the one or more silicone acrylate oligomers or polymers are selected from Formula (I): 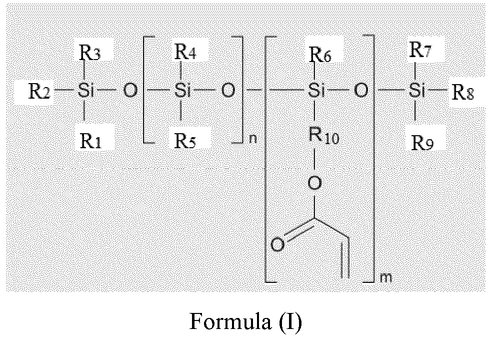 wherein,
R1-R9 are independently selected from -H, a C1-C10 radical, -OH, alkoxy radical, and an acrylate functional group,
R10 is selected from a C1-C10 hydrocarbon;
n is selected from 1 to about 100, such as at least about any of the following: 1, 3, 5, 10, 20, 30, 40, and 50, and/or at most about any of the following: 100, 90, 80, 70, 60, and 50, and
m is selected from 1 to about 100, such as at least about any of the following: 1, 3, 5, 10, 20, 30, 40, and 50, and/or at most about any of the following: 100, 90, 80, 70, 60, and 50.

In accordance with certain embodiments of the invention the acrylate group, for example, from Formula (I) may be a methacrylate group. Additionally or alternatively, one or more of R1-R10 may include an acrylate or methacrylate group.

In accordance with certain embodiments of the invention, the one or more epoxy silicone oligomers or polymers are selected from Formula (II): 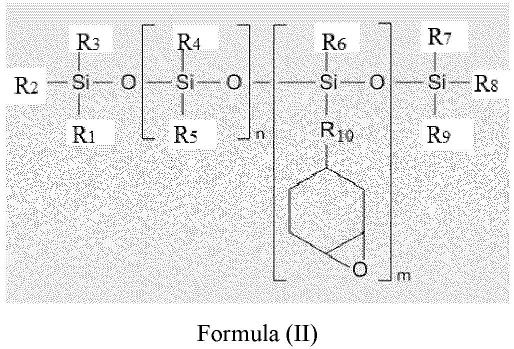 wherein
R1-R9 are independently selected from -H, a C1-C10 radical, -OH, alkoxy radical, and an acrylate functional group,
R10 is selected from a C1-C10 hydrocarbon;
n is selected from 1 to about 100, such as at least about any of the following: 1, 3, 5, 10, 20, 30, 40, and 50, and/or at most about any of the following: 100, 90, 80, 70, 60, and 50, and
m is selected from 1 to about 100, such as at least about any of the following: 1, 3, 5, 10, 20, 30, 40, and 50, and/or at most about any of the following: 100, 90, 80, 70, 60, and 50.

In accordance with certain embodiments of the invention, the NFBC may comprise a coating composition including (a) a wax or a component thereof having an acid value of from 0.1 mg, KOH, g or higher as measured in accordance with Enterprise Standard Test USP 401, such as at least about any of the following: 0.1, 0.5, 0.8, 1, 3, 5, 8, 10, 15, 20, 30, 40, 50, 60, 80, and 100 mg, KOH/g as measured in accordance with Enterprise Standard Test USP 401and/or at most about any of the following: 220, 200, 180, 160, 150, 140, 120, and 100 mg, KOH/g as measured in accordance with Enterprise Standard Test USP 401and (b) a retention aid comprising a nitrogen-containing polymer independently selected from the group consisting of:
(i) a nitrogen-containing polymer of Formula (III), 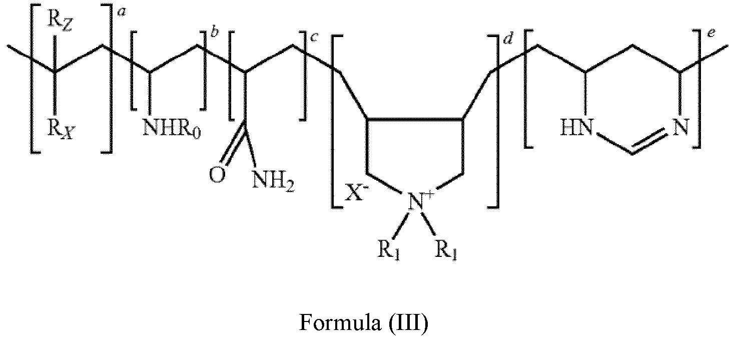
   wherein 'a', 'b, 'c', 'd', and 'e' individually represent the molar percent of each repeating unit included in the nitrogen-containing polymer of Formula (III),
   wherein R₀ is independently selected from the group consisting of: of H, 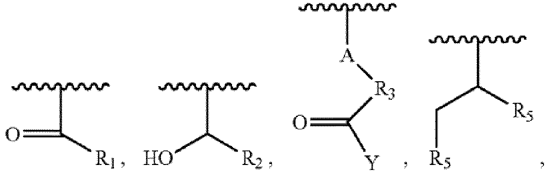 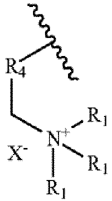 and combinations thereof, and
   wherein:
      R_{z} is independently selected from H, -CH₃, and combinations thereof,
      Rₓ is independently selected from H, -OH, -COOH, -COOR₁, -OCOR₁,-R₁, -R₃OH, -OR₁, -NR₁R₁, -R₃NH₂, -NH₂, -COO(CH₂)₂N(R₁)₂, -COO(CH₂)₂N+(R₁)₃X⁻, -COO(CH₂)₃N+(R₁)₃X⁻, and combinations thereof, with the proviso that when Rₓ is -NH₂, R_{z} is -CH₃,
      Y is independently selected from H, -OH, -R₁, -OR₁, -NR₁R₁, -NH₂, and combinations thereof,
      Z is independently selected from H, -OH, -C=O, -R₁, -OR₁, -NR₁R₁,-NH₂ and combinations thereof,
      R₁ is independently selected from H, a straight chain or branched alkyl or alkenyl containing up to 22 carbons, and combinations thereof,
      R₂ is independently selected from H, a monosaccharide, an oligosaccharide, polysaccharide moiety, a straight or branched alkyl or alkenyl group up to 22 carbons optionally containing a hydroxyl or aldehyde group, and combinations thereof,
      R₃ is independently selected from a straight chain or branched alkyl or alkenyl containing up to 22 carbons or combinations thereof,
      R₄ is independently selected from a straight chain or branched alkyl group containing up to 18 carbons, optionally substituted with a hydroxyl group, and combinations thereof,
      R₅ is independently selected from H, -OH, -COOH, -COOR₁, -OCOR₁,-R₁, -R₁OH, -OR₁, -CONH₂, -CONHCHOHCHO, -NR₁, -NR₁R₁, -R₁NH₂,-NH₂, and combinations thereof,
      A is independently selected from C=O, -CH₂, and combinations thereof, and
      X⁻ is independently an anion;
ii. a polyethyleneimine;
iii. a polyaminoamide;
iv. a copolymer formed from the reaction product of epichlorohydrin and dimethylamine; and
v. combinations thereof.

In accordance with certain embodiments of the invention, 'a', 'b, 'c', 'd', and 'e' of Formula (III) may each independently from each other have a value from 0 to 100 mol.%, such as at least about any of the following: 0, 1, 3, 5 ,8, 10, 15, 20, 25, 30, 35, 40, 45, and 50 mol.%, and/or at most about any of the following: 100, 95, 90, 85, 80, 75, 70, 65, 60, 55, and 50 mol.%.

Persons having ordinary skill in the art understand that many waxes, particularly naturally occurring waxes, include a combination of individual components. For example, naturally occurring beeswax includes palmitate, palmitoleate, and oleate esters of long-chain (e.g. 30-32 carbons) aliphatic alcohols, with each individual component being a "component thereof" in relation to beeswax. For ease of reference, the term "wax or component thereof" may be collectively referred to as "wax" throughout the remaining description.

Although the wax may not be limited to any particular wax in accordance with certain embodiments of the invention, provided the wax has an acid value from 10 mg to 220 mg, KOH/g, typically the wax may be selected from a group consisting of a stearate, beeswax (both synthetic and natural), candelilla wax, palmitate, behenate, and combinations thereof. For example, the wax of the sizing agent may be beeswax or a stearate, or both. Alternatively, the wax may be behenate or palmitate, or both.

In accordance with certain embodiments of the invention, the first nonwoven fabric comprises a basis weight and the alcohol repellant comprises from about 0.1 to about 20% by weight of the basis weight on a dry basis, such as at least about any of the following: 0.1, 0.5, 0.8, 1, 1.5, 2, 2.5, 3, 3.5, 4, 4.5, 5, 5.5, 6, 6.5, 7, 7.5, 8, 8.5, 8, 9.5, and 10% by weight of the basis weight, and/or at most about any of the following: 20, 19, 18, 17, 15, 15, 14, 13, 12, 11, and 10% by weight of the basis weight. For example only, if the first nonwoven has a basis weight of 100 gsm, 20% by weight of the basis weight of the first nonwoven fabric is 20 gsm.

In accordance with certain embodiments of the invention, the barrier fabric may comprise an antistatic composition located on the first outermost surface, the second outermost surface, or both. In accordance with certain embodiments of the invention, the antistatic composition may be located on at least a portion of the first outermost surface and also on at least the second outermost surface. The first outermost surface may have the antistatic composition on one or more separate and discrete locations or, alternatively, be completely coated with the antistatic composition. In accordance with certain embodiments of the invention, the second outermost surface may have the antistatic composition on one or more separate and discrete locations or, alternatively, be completely coated with the antistatic composition. In accordance with certain embodiments of the invention, the NFBC and the antistatic composition may be applied separately to the first nonwoven fabric and/or applied to different regions of the first nonwoven fabric, whether on the same side of the fibrous substrate or different sides of the fibrous substrate. Additionally or alternatively, the NFBC may incorporate the antistatic composition (e.g., formulated as a single composition including the constituents of both the NFBC and the antistatic composition). In this regard, a single composition may provide a method of simultaneously applying a NFBC and an antistatic agent.

In accordance with certain embodiments of the invention, the antistatic composition comprises at least one antistatic agent, in which the antistatic composition comprises at least one of a non-ionic antistatic agent, an anionic antistatic agent, a cationic antistatic agent, an amphoteric antistatic agent, or any combination thereof. In accordance with certain embodiments of the invention, the at least one antistatic agent comprises an alkylphosphate or a phosphate ester.

In accordance with certain embodiments of the invention, the nonwoven fabric has a basis weight and the antistatic composition may comprise from about 0.01 to about 10% by weight (e.g., on a dry basis) of the basis weight, such as at least about 0.01, 0.05, 0.1, 0.25, 0.5, 0.75, 1, 1.5, 2, 2.5, 3, 3.5, 4, 4.5, and 5% by weight (e.g., on a dry basis) and/or at most about any of the following: 10, 9, 8, 7, 6, and 5% by weight (e.g., on a dry basis).

In accordance with certain embodiments of the invention, the NFBC may comprise at least one binder. The at least one binder (if present), for instance, may comprise an anionic binder, a cationic binder, non-ionic binder, an amphoteric binder, or any combinations thereof. The binder, in accordance with certain embodiments of the invention, may comprise binder agents that are self-cross linking chemicals (e.g., self-crosslinking non-ionic binder) that improve barrier properties, particularly when formulated with the NFBC. In this regard, for example, the NFBC may contain one or more binding agents to the fabric via the same application time and/or operation. In accordance with certain embodiments of the invention, the binder may comprise an ethylene vinyl acetate copolymer emulsion, an acrylic emulsion, a vinyl acrylic emulsion, or combinations thereof. For example, the at least one binder may comprise at least one of an acrylic binder, a styrene-butadiene rubber binder, a vinyl copolymer binder, a vinyl acetate binder, an ethylene vinyl acetate binder, a polyvinyl chloride binder, a polyurethane binder, or any combination thereof. In accordance with certain embodiments of the invention, the at least one binder comprises an acrylic binder, such as an anionic acrylic binder, a cationic acrylic binder, or a non-ionic acrylic binder. The binder agent(s), in accordance with certain embodiments of the invention, may comprise from about 0.1 to about 10% by weight of the basis weight of the first nonwoven fabric on a dry basis, such as at least about any of the following: 0.1, 0.5, 0.8, 1, 1.5, 2, 2.5, and 3% by weight of the basis weight of the first nonwoven fabric on a dry basis, and/or at most about any of the following: 10, 9, 8, 7, 6, 5, 4, and 3% by weight of the basis weight of the first nonwoven fabric on a dry basis. For example only, if the first nonwoven has a basis weight of 100 gsm, 5% by weight of the basis weight of the first nonwoven fabric is 5 gsm. In accordance with certain embodiments of the invention, however, the first nonwoven fabric and/or barrier fabric may be devoid of a binder.

In accordance with certain embodiments of the invention, the NFBC may comprise a wetting agent (e.g., one or more surfactants), such as a cationic wetting agent, an anionic wetting agent, a non-ionic wetting agent, or any combination thereof. The wetting agent, for example, may facilitate coating or the individual fibers of the respective nonwoven layers of the first nonwoven fabric. The first nonwoven fabric comprises a basis weight and the wetting agent comprises from about 0.1 to about 5% by weight of the basis weight of the first nonwoven fabric on a dry basis, such as at least about any of the following: 0.1, 0.5, 0.8, 1, 1.5, 2, 2.5, and 3% by weight of the basis weight of the first nonwoven fabric on a dry basis, and/or at most about any of the following: 5, 4, and 3% by weight of the basis weight of the first nonwoven fabric on a dry basis.

The NFBC, in accordance with certain embodiments of the invention, may comprise a first ratio between the alcohol repellant and the binder on a dry basis (alcohol repellant : binder) from about 1:1 to about 20:1, such as at least about any of the following: 1:1, 2:1, 3:1, 4:1, 5:1, 6:1, 7:1, 8:1, 9:1, and 10:1, and/or at most about any of the following: 20:1, 19:1, 18:1, 17:1, 16:1, 15:1, 14:1, 13:1, 12:1, 11:1, and 10:1. Additionally or alternatively, the NFBC comprises a second ratio between the alcohol repellant and the wetting agent on a dry basis (alcohol repellant: wetting agent) from about 1:1 to about 20:1, such as at least about any of the following: 1:1, 2:1, 3:1, 4:1, 5:1, 6:1, 7:1, 8:1, 9:1, and 10:1, and/or at most about any of the following: 20:1, 19:1, 18:1, 17:1, 16:1, 15:1, 14:1, 13:1, 12:1, 11:1, and 10:1. Additionally or alternatively, the NFBC comprises a third ratio between the binder and the wetting agent on a dry basis (alcohol repellant : wetting agent) from about 0.5:1 to about 10:1, such as at least about any of the following: 0.5:1, 0.8:1, 1:1, 1.5:1, 2:1, 2.5:1, 3:1, 3.5:1, 4:1, 4.5:1, and 5:1, and/or at most about any of the following: 10:1, 9:1, 8:1, 7:1, 6:1, and 5:1.

In accordance with certain embodiments of the invention, the first nonwoven fabric comprises a basis weight and the NFBC comprises from about 0.3 to about 35% by weight of the basis weight on a dry basis, such as at least about any of the following: 0.3, 0.5, 0.8, 1, 1.5, 2, 2.5, 3, 3.5, 4, 4.5, 5, 5.5, 6, 6.5, 7, 7.5, 8, 8.5, 8, 9.5, 10, 12, 14, 15, 16, 18, and 20% by weight of the basis weight, and/or at most about any of the following: 35, 32, 30, 28, 26, 25, 24, 22, and 20% by weight of the basis weight.

In accordance with certain embodiments of the invention, a total amount of fine fibers comprising the first plurality of fine fibers and the second plurality of fine fibers account for about 1 to about 40% by weight of the first nonwoven fabric, such as at least about any of the following: 1, 3, 5, 8, 10, 12, 15, 16, 18, 20, 22, 24, 25, 26, 28, and 30% by weight of the first nonwoven fabric, and/or at most about any of the following: 40, 38, 36, 35, 34, 32, and 30 % by weight of the first nonwoven fabric. Additionally or alternatively, a total amount of continuous spunbond fibers comprising the first plurality of continuous spunbond fibers and the second plurality of continuous spunbond fibers account for about 60 to about 99% by weight of the first nonwoven fabric, such as at least about any of the following: 60, 62, 64.65. 66, 68, and 70% by weight of the first nonwoven fabric, and/or at most about any of the following: 99, 97, 95, 92, 90, 88, 85, 84, 82, 80, 78, 76, 74, 72, and 70% by weight of the first nonwoven fabric.

In accordance with certain embodiments of the invention, the first plurality of continuous spunbond fibers, the second plurality of continuous spunbond fibers, or both independently from each other have an average diameter from about 10 to about 25 microns, such as at least about any of the following: 10, 13, 15, and 18 microns, and/or at most about any of the following: 25, 22, 20, and 18 microns. Additionally or alternatively, the first plurality of continuous spunbond fibers, the second plurality of continuous spunbond fibers, or both independently from each other have a round cross-section or a non-round cross-section. For example, at least the first plurality of continuous spunbond fibers, the second plurality of continuous spunbond fibers, or both independently from each other may have from 10 to 100% by number of non-round cross-sectional fibers, such as at least about any of the following: 10, 20, 30, 40, and 50% by number, and/or at most about any of the following: 100, 95, 90, 85, 80, 70, 60, and 50% by number. Additionally or alternatively, at least the first plurality of continuous spunbond fibers, the second plurality of continuous spunbond fibers, or both independently from each other have from 10 to 100% by number of round cross-sectional fibers, such as at least about any of the following: 10, 20, 30, 40, and 50% by number, and/or at most about any of the following: 100, 95, 90, 85, 80, 70, 60, and 50% by number.

In accordance with certain embodiments of the invention, the first plurality of fine fibers, the second plurality of fine fibers, or both independently from each other have an average diameter from about 0.2 to about 8 microns, such as at least about any of the following: 0.2, 0.5, 0.8, 1, 1.5, 1.8, and 2 microns, and/or at most about any of the following: 8, 6, 5, 4.5, 4, 3.5, 3, 2.5 and 2 microns. Additionally or alternatively, the first plurality of fine fibers, the second plurality of fine fibers, or both independently from each other comprise meltblown fibers, melt-fibrillated fibers, or electrospun fibers.

In accordance with certain embodiments of the invention, the fibrous structure may comprise one of the following structures:

(Structure 1) S1ₐ-M_{b}-S2_{c} ;

(Structure 2) S1ₐ-N_{d}-S2_{c} ;

(Structure 3) S1ₐ-M_{b}-N_{d}-S2_{c} ;

(Structure 4) S1ₐ-N_{d}-M_{b}-N_{d}-S2_{c}

(Structure 5) S1ₐ-M_{b}-N_{d}-M_{b}-S2_{c} ;

or any combinations thereof; wherein
'M' comprises a meltblown layer or a melt-fibrillated layer;
'N' comprises a sub-micron fiber-containing layer, such as electrospun fibers;
'S1' comprises a first spunbond layer;
'S2' comprises a second spunbond layer;
'a' represents the number of layers and is independently selected from 1, 2, 3, 4, and 5;
'b' represents the number of layers is independently selected from 1, 2, 3, 4, 5, 6, 7, and 8;
'c' represents the number of layers is independently selected from 1, 2, 3, 4, and 5; and
'd' represents the number of layers is independently selected from 1, 2, 3, 4, 5, 6, 7, and 8;
'e' represents the number of layers is independently selected from 1, 2, 3, 4, and 5.

The first nonwoven fabric, in accordance with certain embodiments of the invention, may have a structure according to Structure 1, and wherein 'a' is 1 or 2, 'b' is 3, 4, or 5, and 'c' is 1 or 2.

The first nonwoven fabric, in accordance with certain embodiments of the invention, may have a hydrohead (e.g., hydrostatic head) from about 30 mbar, such as at least about any of the following: 30, 35, 40, 50, 60, 70, 80, 90, 100, 110, 120, 130, 140, and 150 mbar and/or at most about any of the following: 250, 220, 200, 180, 160, and 150 mbar. Additionally or alternatively, the first nonwoven fabric may have an air permeability of 5 CFM or greater according to IST70.1, such as at least about any of the following: 5, 6, 8, 10, 12, 14, and 15 CFM, and/or at most about any of the following: 30, 28, 25, 22, 20, 18, and 15 according to IST70.1.

In accordance with certain embodiments of the invention, the barrier fabric may have an alcohol repellency rating of at least 5 as determined according to IST 80.8, or at least about 6 as determined according to IST 80.8, or at least about 7 as determined according to IST 80.8 or at least about 8 as determined according to IST 80.8. Additionally or alternatively, the barrier fabric may have a static decay of from about 0.01 to about 0.5 seconds as tested according to IST 40.2 performed at 50% R.H. and using 10% remaining charge as the cut-off level, such as at least about any of the following: 0.01, 0.02, 0.05, 0.08, and 0.1 seconds and/or at most about any of the following: 5, 4, 3, 2, 1.5, 1.2, and 1 seconds. Additionally or alternatively, the barrier fabric may have a static decay of from about 1 to about 3 seconds as tested according to IST 40.2 performed at 30% R.H. and using 10% remaining charge as the cut-off level, such as at least about any of the following: 1, 1.2, 1.4, 1.6, 1.8, and 2 seconds and/or at most about any of the following: 3, 2.8, 2.6, 2.4, 2.2 and 2 seconds.

In accordance with certain embodiments of the invention, the barrier fabric may have a basis weight from about 10 to about 280 gsm, such as at least about any of the following: 10, 15, 20, 25, 30, 34, 40, 45, 50, 60, 80, and 100 gsm, and/or at most about any of the following: 280, 270, 250, 220, 200, 180, 150, 120, and 100 gsm.

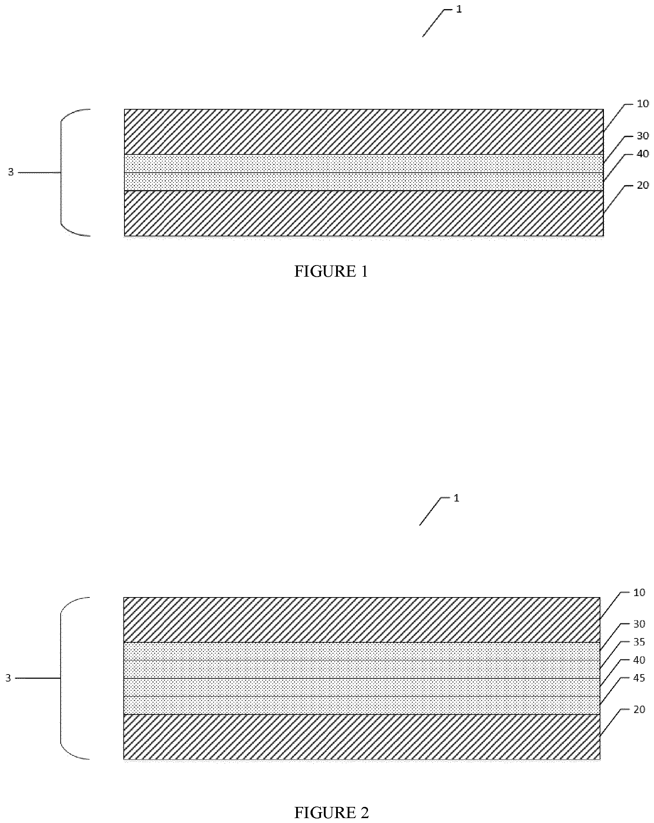

Figure 1, for instance, illustrates a cross-sectional view of a barrier fabric 1 according to certain embodiments of the invention, in which the barrier fabric includes a first outermost spunbond layer 10 and a second outermost spunbond layer 20. This particular example also includes a first inner fine fiber-containing nonwoven layer 30, such as a meltblown layer, and a second inner fine fiber-containing nonwoven layer 40. Figure 2 illustrates a cross-sectional view of another barrier fabric 1 according to certain embodiments of the invention. In this particular example, the barrier fabric includes four (4) inner fine fiber-containing nonwoven layers 30,35,40,45, such as four meltblown layers.

In another aspect, the present invention provides an underpad for a low air loss bed. The underpad may comprise a backsheet including or consisting of a barrier fabric according to any of those described and disclosed herein, a liquid permeable topsheet, an absorbent core located between the backsheet and the liquid permeable topsheet. In accordance with certain embodiments of the invention, the backsheet and/or the underpad is devoid of a film layer.

In another aspect, the present invention provides a method of forming a barrier fabric, in which the method may include the following steps: (i) providing or forming a first outermost spunbond layer including a first plurality of continuous spunbond fibers; (ii) providing or forming a second outermost spunbond layer including a second plurality of continuous spunbond fibers; (iii) providing or forming a plurality of inner fine fiber-containing nonwoven layers including a first fine fiber-containing layer including a first plurality of fine fibers and a second fine fiber-containing layer including a second plurality of fine fibers; (iv) positioning the plurality of inner fine fiber-containing nonwoven layers directly or indirectly between the first outermost spunbond layer and the second outermost spunbond layer; and (v) topically applying a non-fluorinated barrier coating (NFBC) composition on each of the first outermost spunbond layer, the second outermost spunbond layer, the first fine fiber-containing layer, and the second fine fiber-containing layer.

In accordance with certain embodiments of the invention, each of the first outermost spunbond layer, the second outermost spunbond layer, the first fine fiber-containing layer, and the second fine fiber-containing layer are individually subjected to the step of topically applying the NFBC. For example, each and every individual layer of the first nonwoven fabric may be topically treated or coated with the NFBC. For example, each of the individual nonwoven layers may be provided separately and being pre-coated with the NFBC and subsequently assembled and consolidated together to form the barrier fabric. Alternatively, each of the individual nonwoven layers may be produced in a multi-beam manufacturing line to form an intermediate material (e.g., multi-layer nonconsolidated nonwoven), subjecting the intermediate material to coating with the NFBC, and consolidation of the intermediate material. Additionally or alternatively, the intermediate material may be consolidated and then subjected to coating with the NFBC. In accordance with certain embodiments of the invention, the individual layers may be individually coated during the laydown of the fibers forming each individual layer and/or subsequent to the laydown of a given layer and prior to the laydown of the next nonwoven layer.

In accordance with certain embodiments of the invention, the step of topically applying the NFBC composition may comprise a spraying operation, a roller coating operation, such as kiss-coating, an offset gravure coating operation, or saturation by pool submersion.

In accordance with certain embodiments of the invention, the method comprises a step of consolidating the first outermost spunbond layer, the second outermost spunbond layer, the first fine fiber-containing layer, and the second fine fiber-containing layer together. The step of consolidation comprises a thermal bonding operation or ultrasonic bonding operation, which forms a plurality of discrete bond sites defining a bonded area. For example, the bonded area comprises from about 8 to about 40%, such as at least about any of the following: 8, 10, 12, 15, 18, 20, 22, and 25%, and/or at most about any of the following: 40, 35, 30, 28, and 25%.

### Examples

The present disclosure is further illustrated by the following examples, which in no way should be construed as being limiting. That is, the specific features described in the following examples are merely illustrative and not limiting.

### EXAMPLE SET 1

### A. Test Methods

Basis weight of the following examples was measured according to ASTM test method D3776. The results were provided in units of mass per unit area in g/m² (gsm).

Alcohol repellency of the following examples was measured according to test method IST 80.8

Hydrohead of the following examples was measured according to standard test method IST 80.8 and ramping up the pressure at a rate of 60 mbar/min. A larger hydrohead value is more desirable for increased the barrier performance.

Air Permeability is a measure of air flow passing through a sheet under at a stated pressure differential between the surfaces of the sheet and was conducted according to ASTM D 737, Test area 38cm², Test Pressure @ 125 Pa, and is reported in ml/dm²/min. A larger air permeability value is indicative of improved comfort for surgical gown and drape applications.

Low Surface Tension Strikethrough Time (LSTST) is a test that determines the time it takes for a particular quantity of liquid discharged at a prescribed rate to fully penetrate a sample of a nonwoven fabric. The method employed herein is a modification to WSP 70.3 (05). The changes were as follows: the test liquid was a 32 mN/m surface tension liquid prepared with Triton-X-100 and distilled water.

### B. General Manufacturing Method

All base nonwoven samples were made from a polypropylene SMS nonwoven comprising at least a layer of polypropylene meltblown fibers positioned between at least two layers of polypropylene continuous spunbond fibers and point bonded using a hot calender. More specifically the inventive sample base materials were SMMMS or SMMMMS structures made on a 5 or 6 beams production line known as a Reicofil 4 at similar speed and process conditions. The process consisted of forming a plurality of continuous spunbond filaments from a first bean (e.g., beam 1) that are deposed on a foraminous moving surface, then using Beams 2, 3, 4 (e.g., for a three layer meltblown structure) or Beams 2, 3, 4, 5 (e.g., for a four layer meltblown structure) for forming polypropylene meltblown fibers that are deposited on top of the layer of continuous spunbond filaments from Beam 1. Subsequently, an additional beam (e.g., Beam 5 or Beam 6) is used to spin continuous spunbond filaments that are deposited on top of the meltblown layers to form an intermediate nonowoven composite web. The intermediate composite web is then fed to the nip point of a calender where it was point bonded under a pressure 950 N/cm and 160°C temperature. The bonding pattern occupied about 18% of the nonwoven surface. The bond points had an elliptical shape. Inventive Examples identified as Example 3, for example, was manufactured in this method. Inventive Example 8, however, was a hydroentagled composite of wood pulp with synthetic fibers. Inventive Examples 10 and 12 were 100% PET spunlace fabrics.

Table 1 below provides a summary of the Test Result Data for Inventive Examples identified as Examples 3, 8, 10, 12, 14, which were coated with a NFBC and the Comparative Examples are identified as Examples 1, 2, 4, 5, 6, 7, 9, 11, 13, which were either untreated or C6FC treated or a breathable film as identified in Table 1.

With regard to the Inventive Examples, Example 3 was a 44 gsm SMMMS structure that was hand treated with ~ 1.5 % active add-on (e.g., dry basis) of the NFBC. This example achieved an average Liquid Strike Through Time (LSTST) of 529 seconds (up to 722 seconds), which compared reasonably well to the C6FC coated nonwoven of Example 1, which had the same basis weight. Example 3 utilized a 1 wt.% on a dry basis of a wetting agent and 7 wt.% on a dry basis of a cationic non-fluorochemical agent, such as those described and disclosed herein.

Example 8 was a 75 gsm EFP (e.g., hydroentangled composite of wood pulp and polypropylene continuous fibers) treated with ~ 1.7 % active add-on (e.g., dry basis) of the NFBC. This example achieved an average LSTST of 2372 seconds (up to 3252 second), a hydrohead of 27 mbar, an IPA 40% repellency and Spray Impact < 0.1g per AATCC 42. These performance properties are similar to C8 or C6 FC chemical treated EFP material for surgical gowns or drapes. Example 8 utilized 8 wt.% of a cationic non-fluorochemical agent, such as those described and disclosed herein.

Examples 10 (30 gsm) and 12 (40 gsm) were formed from 100% PET Spunlace base made in Berry Europe -Ostomy Nonwoven and treated with ~ 1.7 % active add-on (dry basis) of the NFBC. Each of these examples achieved average hydrohead of around 11 mbar and an LSTST 8.4 seconds. These performance properties met Berry Europe -Ostomy C6FC treated nonwoven requirement. Examples 10 and 12 utilized 8 wt.% of a cationic non-fluorochemical agent, such as those described and disclosed herein.

Additionally, the Inventive Examples also passed the blood drop test with synthetic blood in accordance with ASTM F1670M (e.g., 15 minutes droplets exposure without blood penetration).

In Example 14, a 13 gsm SMS nonwoven fabric was constructed that had 16% by weight of meltblown fibers (AMB 1.5), in which the SMS was treated with ~ 1.5 % active add-on (dry basis) of a NFBC. This example achieved an average LSTST of 35 second, which is comparable to fluorine treated diaper back sheet in the current market.

**Table 1**

| **Sample ID** | **Hydro Static Head (60mbar)** | **LSTST** | **Max IPA Repellency** | **BW** | **Thickness** | **AP** | **AP** |
|---|---|---|---|---|---|---|---|
| | 3rd drop | second | rating | gsm | microns | CFM | m3/m2/min |
| | | | | | | | |
| **(1) 44 gsm SMMMS untreated made on SZ 51 China** | 45 | 20.34 | 2 | 43.51 | 319.00 | 46.33 | 14.13 |
| **(2) 44 gsm SMMMMS C6FC AR/AS treated (SZT2A215993502 )** | 77 | 942 | 8 | 44.73 | 356.67 | 32.63 | 9.94 |
| **(3) LW722-A-1 /2 (44gsm SMMMS hand treated with Non-FC Example A** | | 529 | 4 | 44 | | | |
| **(4) 16 gsm BR172 for Cardinal breathable film** | 57 | >1800 | | 16.20 | 23.33 | 0.01 | 0.00 |
| **(5) 69 gsm SMMMMS C6FC AR/AS treated : SZT2D216506703 (for Cardinal underpad code SCD4959F)** | 134 | 576 | 8 | 72.21 | 470.00 | 13.50 | 4.11 |
| **(6) 70gsm SMMMMS untreated : SZTD911612103** | 88 | 69 | 2 | 70.64 | 455.00 | 15.50 | 4.72 |
| **(7) 60gsm SMMMMS untreated : SZTD911611503** | 119 | 144 | 2 | 60.50 | 428.67 | 20.47 | 6.25 |
| **(8) EFP treated with Non-FC Example A sample** | 27 | **2372** | 4 | 75 | 323 | 54.9 | 16.7 |
| **(9) 30gsm 100% PET Spunlace base made in Berry Eumpe-Ostomy Nonwoven** | 9.2 | 46 | 1 | 30 | 444 | 662 | 202 |
| **(10) 30gsm 100% PET Spunlace base made in Berry Europe-Ostomy Nonwoven treated with Non-FC Example A** | 11.2 | 8.43 | 4 | 30.5 | | | |
| **(11) 40gsm 100% PET Spunlace base made in Berry Europe-Ostomy Nonwoven** | 8.3 | 4.05 | 1 | 40 | 454 | 500 | 153 |
| **(12) 40gsm 100% PET Spunlace base made in Berry Europe-Ostomy Nonwoven treated with Non-FC Example A** | 10.7 | 8.34 | 4 | 40.5 | | | |
| **(13) 13gsm Hygiene with 16% MB [AMB 1.5]** | 18.79 | 11.27 | | 13 | | | 42.81 |
| **(14) 13gsm Hygiene with 16% MB [AMB 1.5] treated with Non-FC Example A** | | 535 | 3 | 13 | | | |

### EXAMPLE SET 2

An additional set of examples were conducted and tested for various physical properties. For example, a 45gsm PP SMMMS KamiSoft material and 44gsm, 69gsm PP SMMMMS nonwovens were produced and coated with Non-FC Example A plus a higher percentage of binder compared to that of Examples 8, 11 and 13 from Example Set 1. Test results are listed in Table 2 below. These coated samples with higher % binder (e.g., on a dry basis) have 1.76 wt% solid Non-FC Example A chemical with 1.6 wt% binder (ration on a dry basis of Non-FC Example A (excluding binder) to binder of 1.1:1). The results show that the Ethanol Alcohol repellency achieved up to 70% repellency with the test method IST 80.8. Also the treated nonwovens maintained MD tensile strength after finishing process (tested by Berry MONC lab Test Code 56: pull speed 30 cm/min, jaw separation of 10 cm, sample size of 50 mm x 175 mm, grip size of 50 mm, and units in N/5cm). The static decay was tested using the standard test method IST 40.2 performed at 50% RH using 50% remaining charge as cut-off level. The properties of the resulting fabrics ( Examples 8, 11 and 13 ) are summarized in Table 2 below. A static decay at 50% RH with 50% cut-off need to be less than 1 second with Target 0.5 second.

The binder used was a self-crosslinking non-ionic acrylic binder emulsion, and the observed IPA (Isopropanol repellent) rating improved from 4 to 5 ( 40% to 50% IPA repellent) and Ethanol repellency rating improved from 5 to 7 ( 50% to 70% IPA repellent) by add higher amount of binder. Additionally, 0.8% ~ 1.6% by weight on a dry basis of non-FC formula was added for 30gsm, 40gsm 100% PET Spunlace nonwoven and 40gsm PE spunbond , PP SMMMMS nonwoven.

The Sample (2) 40gsm PE spunbond (medical Ostomy nonwoven) treated with ~ 1.32 % dry weight add-on (= 1.32 wt% ) Non-FC Example A plus 1.2% Binder achieved IPA 50% repellency and 70% Ethanol Alcohol repellency. Example 2 utilized a 1.2 wt.% of a wetting agent, 6 wt.% of a cationic non-fluorochemical agent, such as those described and disclosed herein, and 3 wt.% of binder, as noted below.

The Sample (4), (6) 30gsm & 40gsm 100% PET Spunlace (medical Ostomy nonwoven) treated with ~ 1.32 % dry weight add-on (= 1.32 wt% ) Non-FC Example A plus 0.8% Binder achieved IPA 50% repellency and 70% , 80% Ethanol Alcohol repellency. These examples utilized a 1.2 wt.% of a wetting agent, 6 wt.% of a cationic non-fluorochemical agent, such as those described and disclosed herein, and 2 wt.% on a dry basis of binder, as noted below.

The Samples (8), (11), and (23) were 45 gsm, 44 gsm, and 69 gsm polypropylene SMMMS nonwovens, respectively. Each Sample utilized a 1.6 wt.% of a wetting agent, 8 wt.% of a cationic non-fluorochemical agent, such as those described and disclosed herein, and 4 wt.% of binder, as noted below.

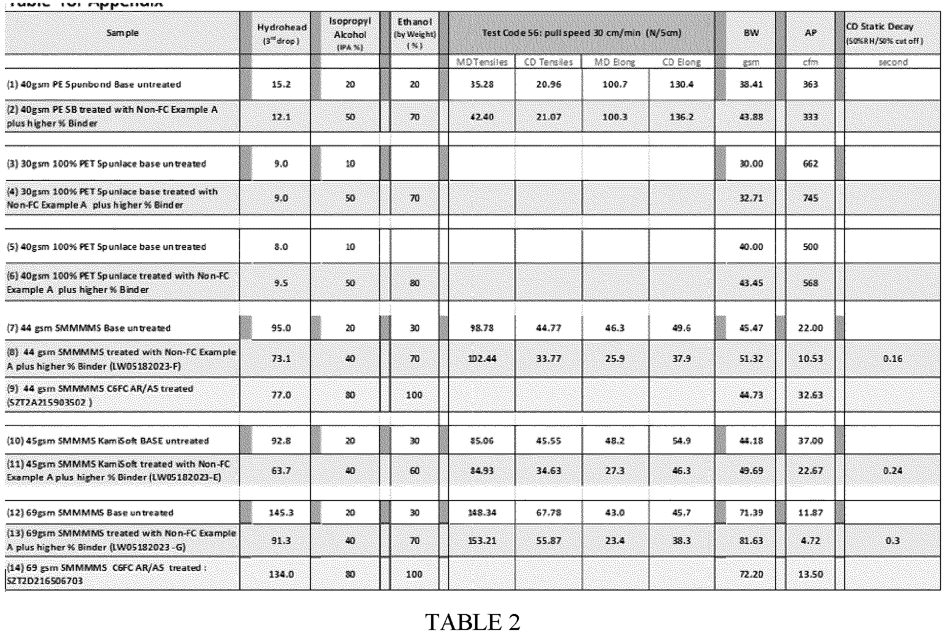

In the foregoing inventive examples, the Non-FC Example A was a cationic acrylic based polymer, containing an alkyl silane methacrylate group. The wetting agent mentioned above was Alkanol^{™} 6112 (Decan-1-ol; CAS No. 112-30-1).

### Example 3 - SMMMMS Fabric with Surface treatment

An additional set of Examples were performed by bath padding a 44gsm, a 22gsm, and a 69gsm polypropylene SMMMMS nonwoven fabric coated with Non-FC Example A, which was a cationic acrylic based polymer contained an alkyl silane methacrylate group) plus a higher percentage of binder in some examples (e.g., Examples 3, 6 and 13 as shown in Table 3). The binder used was a self-crosslinking non-ionic acrylic binder emulsion. The test result are listed in Table 3, which is provided below.

Of note, the coated samples having a higher % binder had 2.2 wt % solid of Non-FC Example A with 2 wt. % solids of the binder. As noted above Examples 3, 6, and 13 included the higher wt. % of binder in the topical treatment. These Examples (i.e., Examples 3, 6, and 13) included the following components in the bath padder from which the Examples were coated (e.g., surface treated): 3 wt.% of a wetting agent (i.e., Alkanol^{™} 6112 (Decan-1-ol; CAS No. 112-30-1); 10 wt.% Cationic non-FC Example A; 5 wt.% of the binder. We observed that the IPA (Isopropanol repellent) rating improved from 4 to 5 ~ 6 ( 40% to 50% ~ 60% IPA repellent) and the Ethanol Alcohol repellency achieved up to 70% repellency according to test method IST 80.8.

Example 5, which was a 22 gsm polypropylene SMMMMS nonwoven fabric, was treated with ~ 1.32 % dry weight add-on (= 1.32 wt% ) Non-FC Example A plus 2 wt.% binder and achieved IPA 40% repellency and 70% Ethanol Alcohol repellency with higher LSTST 2700 sec. Example 5 included the following components in the bath padder from which Example 5 was coated (e.g., surface treated): 3 wt.% of a wetting agent (i.e., Alkanol^{™} 6112 (Decan-1-ol; CAS No. 112-30-1); 6 wt.% Cationic non-FC Example A; 5 wt.% of the binder.

Example 12, which was a 69gsm polypropylene SMMMMS nonwoven fabric, was treated with 2.2 % dry weight add-on (= 2.2 wt% ) Non-FC Example A plus 1.2 % solid of binder. Example 12 included the following components in the bath padder from which Example 12 was coated (e.g., surface treated): 2 wt.% of a wetting agent (i.e., Alkanol^{™} 6112 (Decan-1-ol; CAS No. 112-30-1); 10 wt.% Cationic non-FC Example A; 3 wt.% of the binder.

As noted above, the binder was a self-crosslinking non-ionic acrylic binder emulsion and observed IPA (Isopropanol repellent) rating from 4 to 5 ~ 6 ( 40% to 50% ~ 60% IPA repellent ) and Ethanol repellency rating from 5 to 7 ( 50% to 70% IPA repellent) was realized by incorporating a higher amount of the binder. In this regard, 1.2% ~ 2 % dry weight add-on of the binder by weight of the fabric into non-FC formula provided noticeable improvements.

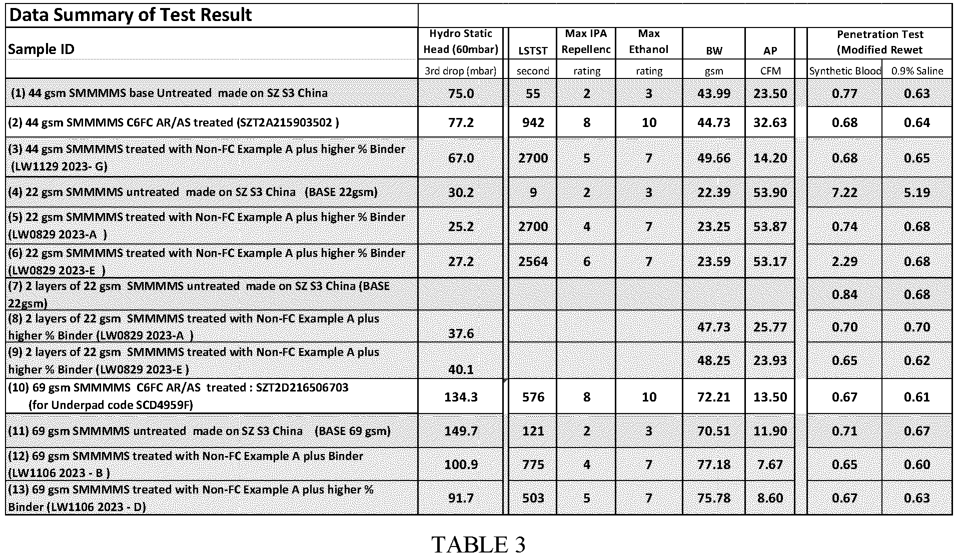

These and other modifications and variations to the invention may be practiced by those of ordinary skill in the art without departing from the spirit and scope of the invention, which is more particularly set forth in the appended claims. In addition, it should be understood that aspects of the various embodiments may be interchanged in whole or in part. Furthermore, those of ordinary skill in the art will appreciate that the foregoing description is by way of example only, and it is not intended to limit the invention as further described in such appended claims. Therefore, the spirit and scope of the appended claims should not be limited to the exemplary description of the versions contained herein.

## Claims

1. A barrier fabric, comprising: a first nonwoven fabric comprising -
(i) a first outermost spunbond layer including a first plurality of continuous spunbond fibers;
(ii) a second outermost spunbond layer including a second plurality of continuous spunbond fibers;
(iii) a plurality of inner fine fiber-containing nonwoven layers including a first fine fiber-containing layer including a first plurality of fine fibers and a second fine fiber-containing layer including a second plurality of fine fibers, wherein the plurality of inner fine fiber-containing nonwoven layers are located directly or indirectly between the first outermost spunbond layer and the second outermost spunbond layer; and
(iv) a non-fluorinated barrier coating (NFBC) located on each of the first outermost spunbond layer, the second outermost spunbond layer, the first fine fiber-containing layer, and the second fine fiber-containing layer.

2. The fabric of claim 1, wherein the NFBC comprises an alcohol repellant comprising an acrylic-functional polymer including an alkyl silane methacrylate group.

3. The fabric of claim 2, wherein the acrylic-functional polymer is cationic.

4. The fabric of claim 1, wherein the first nonwoven fabric comprises a basis weight and an alcohol repellant that comprises from about 0.1 to about 20% by weight of the basis weight on a dry basis.

5. The fabric of claim 2, wherein the NFBC comprises a binder, wherein the binder comprises an anionic binder, a cationic binder, non-ionic binder, an amphoteric binder, or any combinations thereof.

6. The fabric of claim 5, wherein the binder comprises a crosslinked binder.

7. The fabric of claim 5, wherein the first nonwoven fabric comprises a basis weight and the binder comprises from about 0.1 to about 10% by weight of the basis weight on a dry basis.

8. The fabric of claim 2, wherein the NFBC comprises a wetting agent, and the first nonwoven fabric comprises a basis weight and the wetting agent comprises from about 0.1 to about 5% by weight of the basis weight on a dry basis.

9. The fabric of claim 2, wherein the NFBC comprises a first ratio between the alcohol repellant and a binder on a dry basis (alcohol repellant : binder) from about 1:1 to about 20:1.

10. The fabric of claim 2, wherein the NFBC comprises a second ratio between the alcohol repellant and a wetting agent on a dry basis (alcohol repellant : wetting agent) from about 1:1 to about 20:1.

11. The fabric of claim 1, wherein the first plurality of fine fibers, the second plurality of fine fibers, or both independently from each other have an average diameter from about 0.2 to about 8 microns.

12. The fabric of claim 1, wherein the first nonwoven fabric comprises one of the following structures:
(Structure 1) S1ₐ-M_{b}-S2_{c} ;
(Structure 2) S1ₐ-N_{d}-S2_{c} ;
(Structure 3) S1ₐ-M_{b}-N_{d}-S2_{c} ;
(Structure 4) S1ₐ-N_{d}-M_{b}-N_{d}-S2_{c}
(Structure 5) S1ₐ-M_{b}-N_{d}-M_{b}-S2_{c} ;
or any combinations thereof; wherein
'M' comprises a meltblown layer or a melt-fibrillated layer;
'N' comprises a sub-micron fiber-containing layer, such as electrospun fibers;
'S1' comprises a first spunbond layer;
'S2' comprises a second spunbond layer;
'a' represents the number of layers and is independently selected from 1, 2, 3, 4, and 5;
'b' represents the number of layers is independently selected from 1, 2, 3, 4, 5, 6, 7, and 8;
'c' represents the number of layers is independently selected from 1, 2, 3, 4, and 5; and
'd' represents the number of layers is independently selected from 1, 2, 3, 4, 5, 6, 7, and 8;
'e' represents the number of layers is independently selected from 1, 2, 3, 4, and 5.

13. The fabric of claim 12, wherein the first nonwoven fabric has a structure according to Structure 1, and wherein 'a' is 1 or 2, 'b' is 3, 4, or 5, and 'c' is 1 or 2.

14. An underpad for a low air loss bed, comprising:
(i) a backsheet comprising a barrier fabric according to claim 1, wherein the backsheet is devoid of a film;
(ii) a liquid permeable topsheet; and
(iii) an absorbent core located between the backsheet and the liquid permeable topsheet.

15. A method of forming a barrier fabric, comprising:
(i) providing or forming a first outermost spunbond layer including a first plurality of continuous spunbond fibers;
(ii) providing or forming a second outermost spunbond layer including a second plurality of continuous spunbond fibers;
(iii) providing or forming a plurality of inner fine fiber-containing nonwoven layers including a first fine fiber-containing layer including a first plurality of fine fibers and a second fine fiber-containing layer including a second plurality of fine fibers,
(iv) positioning the plurality of inner fine fiber-containing nonwoven layers directly or indirectly between the first outermost spunbond layer and the second outermost spunbond layer;
(v) topically applying a non-fluorinated barrier coating (NFBC) composition on each of the first outermost spunbond layer, the second outermost spunbond layer, the first fine fiber-containing layer, and the second fine fiber-containing layer;
wherein optionally (i) each of the first outermost spunbond layer, the second outermost spunbond layer, the first fine fiber-containing layer, and the second fine fiber-containing layer are individually subjected to the step of topically applying the NFBC or (ii) each of the first outermost spunbond layer, the second outermost spunbond layer, the first fine fiber-containing layer, and the second fine fiber-containing layer define an intermediate material, and the intermediate material is subjected to the step of topically applying the NFBC.
